# EUROPEAN PATENT SPECIFICATION

(11) **EP 0 579 096 B1**
(45) Date of publication and mention of the grant of the patent: **26.05.1999**
(21) Application number: 93110843.5
(22) Date of filing: 07.07.1993
(51) Int. Cl.: C07D 215/22

(54) **A process for the preparation of 5-(3-tert.-butylamino-2-hydroxypropoxy)-3,4-dihydro- carbostyryl.**
Verfahren zur Herstellung von 5-(3-tert.-butylamino-2-hydroxypropoxy)-3,4-dihydrocarbostyryl
Procédé pour la préparation de 5-(3-tert.-butylamino-2-hydroxypropoxy)-3,4-dihydrocarbostyryl

(30) Priority: 17.07.1992 ES 9201501
(43) Date of publication of application: 19.01.1994
(73) Proprietor: LUSOCHIMICA S.P.A., I-22050 Lomagna (IT)
(72) Inventor: Manghisi, Elso, I-22050 Lomagna (Como) (IT); Perego, Bruno, I-22050 Lomagna (Como) (IT); Salimbeni, Aldo, I-22050 Lomagna (Como) (IT)
(74) Representative: Minoja, Fabrizio, Dr.

(56) References cited:
- FR-A- 2 719 715
- CHEMICAL ABSTRACTS, vol. 82, no. 23, 9 June 1975, Columbus, Ohio, US; abstract no. 156122v, page 590 ;column R ; & JP-A-7 494 684 (OTSUKA PHARMACEUTICAL CO.)
- CHEMICAL ABSTRACTS, vol. 88, no. 9, 27 February 1978, Columbus, Ohio, US; abstract no. 62304c, page 379 ;column L ; & JP-A-7 739 830 (OTSUKA PHARMACEUTICAL CO.)
- CHEMICAL ABSTRACTS, vol. 85, no. 21, 22 November 1976, Columbus, Ohio, US; abstract no. 159893v, page 531 ;column L ; & JP-A-7 648 674 (OTSUKA PHARMACEUTICAL CO.)
- CHEMICAL ABSTRACTS, vol. 88, no. 3, 16 January 1978, Columbus, Ohio, US; abstract no. 22665q, page 614 ;column L ; & JP-A-77 108 980 (OTSUKA PHARMACEUTICAL CO.)
- CHEMICAL ABSTRACTS, vol. 86, no. 1, 3 January 1977, Columbus, Ohio, US; abstract no. 5328y, page 460 ;column L ; & JP-A-7 648 676 (OTSUKA PHARMACEUTICAL CO.)

## Description

The present invention relates to an improved process for the preparation of S5-(3-tert.-butylamino-2-hydroxypropoxy)-3,4-dihydrocarbostyryl of formula (I):

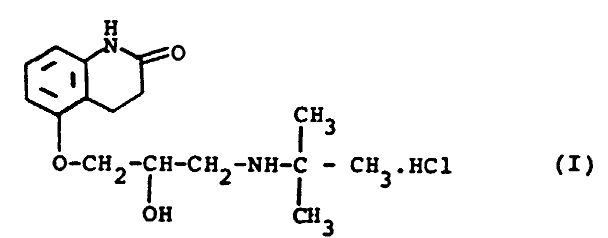

This compound, also known under the international common name Carteolol, is of great practical importance due to its remarkable pharmacological activity. Processes for the preparation of compound (I) are described in literature.

Chemical Abstracts - Vol. 82, NO. 82, NO. 23, 9 June 1975, Columbus - Ohio (USA), abstract NO. 156122v, page 590, column R - describes the synthesis of hydroxylated 3,4-dihydrocarbostyryl derivatives based on the reaction with 1-chloro-2-hydroxy-3-amino-propan.

Chemical Abstracts - Vol. 88, NO. 9, 27 February 1978, Columbus - Ohio (USA), abstact NO. 62304c, page 379, column L - describes a process in which the hydroxy -3,4-carbostyryl is reacted with an azetidinol.

Chemical Abstracts - Vol. 85, NO. 21, 22 November 1976, Columbus - Ohio (USA); abstract NO.159893v, page 531, column L, and vol. 86, NO.1, 3 January 1977, Columbus - Ohio (USA), abstract NO. 5328y, page 460, column L describe processes for the preparation of 8-substituted carbostyryl derivatives.

Chemical Abstracts , Vol. 88, NO. 3, 16 January 1978, Columbus Ohio, (USA), abstract NO. 22665q, page 614, column L - discloses the preparation of (substituted) amino-propanoloxycarbostyryls.

In particular, in ES-A-412,484 (corresponding to FR-A-2 719 715), a method is described in which 5-hydroxy-3,4-dihydrocarbostyryl of formula (II) 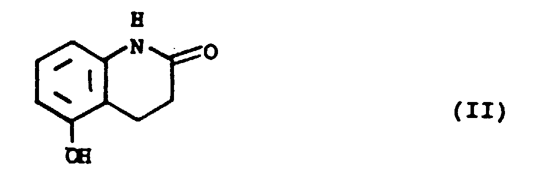 is reacted with an excess of 1 to 10 times on theoretical of a derivative of formula (III) in which X has different meanings and in particular X is Cl: to afford 5-(2,3-epoxy)propoxy-3,4-dihydrocarbostyryl of formula (IV):

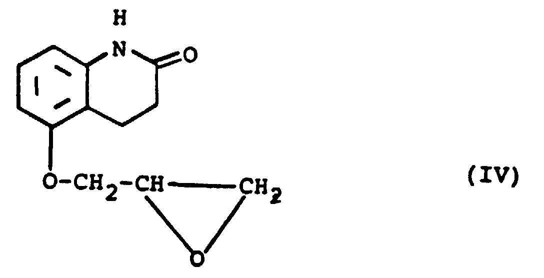

This reaction takes place in the presence of a basic compound (such as alkali metals, hydroxides or carbonates, or organic bases), in the presence or in the absence of inert solvents, such as lower aliphatic alcohols, water, lower alkyl acetates and lower ketones. Preferably, alcohols such as methanol, ethanol, isopropanol and butanol; esters such as ethyl acetate, methyl acetate and propyl acetate; ketones such as acetone and methyl ethyl ketone, are used.

The reaction temperature ranges from 0°C to the boiling point of the solvent used, preferably from 50°C to the boiling point of the solvent, and the reaction time is from 4 to 6 hours.

The yield cited in the mentioned patent ranges between 39% in Example 1, 57% in Example 2, 49% in Example 71 and 54% in Example 73.

No data are given about the purity of the obtained 5-(2,3-epoxy)propoxy-3,4-dihydrocarbostyryl (IV). Subsequently, compound (IV) is reacted with tert-butylamine in a proportion from equimolecular to 8-fold excess on the theoretical amount, in the presence or in absence of inert organic solvents.

Suitable inert solvents are lower alkyl ethers, such as diethyl ether, methyl ethyl ether, dioxane and tetrahydrofuran; hydrocarbons, such as benzene, toluene, xylene; lower aliphatic alcohols, such as methanol, propanol, butanol, etc.; water and dialkylformamides, such as dimethylformamide and the like.

The reaction temperature can range between room temperature and the boiling temperature of the solvent (preferably between 40° and 60°C), the pressure between 1 and 10 atmospheres and the time between 3 and 8 hours, depending on the reaction temperature (preferably from 4 to 5 hours).

The resulting yield is 75% in Example 76 for Carteolol maleate.

Another method is based on the reduction of 5-(2-hydroxy-3-tert-butylaminopropoxy)carbostyryl (V) to afford directly Carteolol (I)

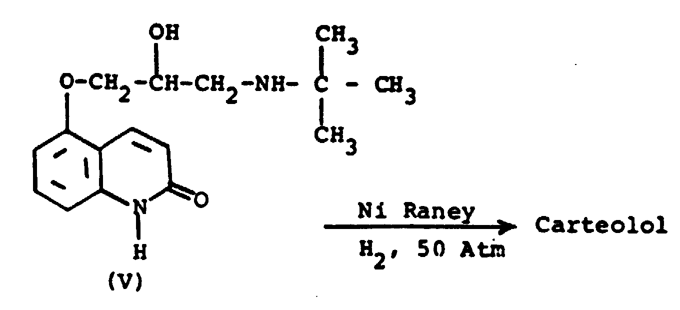

A good yield (94% on a theoretical basis) is obtained, but the preparation of compound (V) is rather laborious.

The preparation of compound (V) is described in the subsequent ES-A-421,430, in which a process is claimed which is characterized by reacting a carbostyryl of formula (VI) 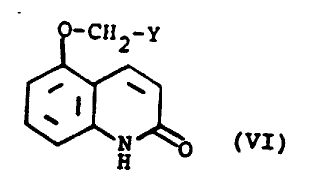 wherein Y is a 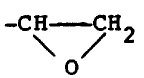 group or a 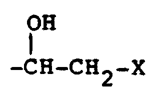 group (X is halogen) with tert-butylamine, in a 66% yield (see Example 2).

The compound of formula (VI) 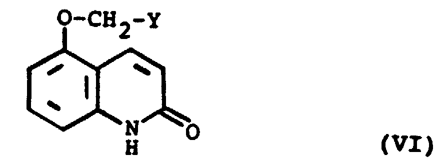 is obtained by condensation of the phenol (VII) 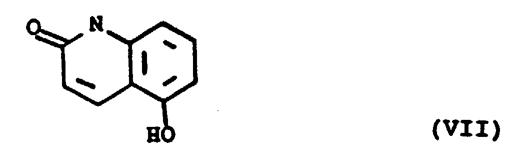 with a compound of formula (III) 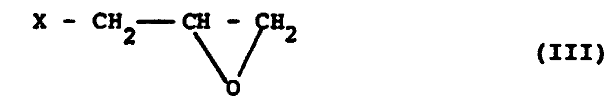 by a similar method to that described for the saturated analogue and the yield is not indicated (Example 1 of the mentioned patent), since the 5-(2-hydroxy-3-chloro) propoxycarbostyryl derivative is formed together with the mentioned product, which is separately isolated. Both compounds are subsequently reacted with tert.-butylamine (yield not indicated).

The analysis of the methods described above clearly shows that the best process for the industrial production is the one schematized below:

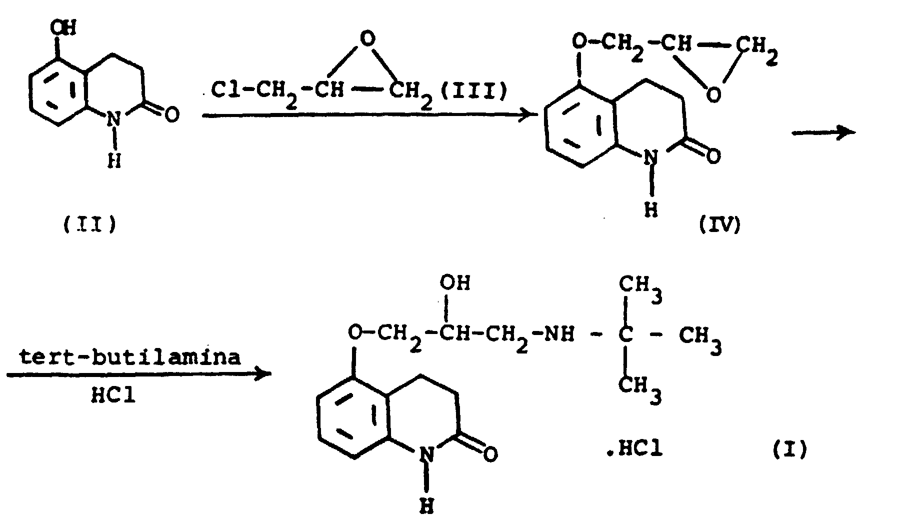

The yield of the first step is about 50% (39 to 57%) and that of the second step around 75%, so the global yield is 37% on the theoretical yield.

Now it has surprisingly been found that the reaction between compounds (II) and (III) takes place with much higher yields and at room temperature (15 - 30°C) when miscible aprotic solvents selected from dimethylsulphoxide, dimethylformamide and methylpyrrolidone and water in certain proportions are used, in the presence of alkali hydroxides. The solvent:water ratio ranges between 1: 0.2 and 1: 1, preferably between 1: 0.3 and 1: 0.6.

The yield ranges from 80 to 85% and the resulting compound (IV) is sufficiently pure to be directly used in the subsequent step, which consists in reacting compound (IV) with tert-butylamine in the presence of dimethylsulphoxide at a temperature between 50° and 80°C. The yield of this step is about 80%, affording a product of satisfactory purity.

The advantages of the process of the invention are the following:
1) increase in the yield, which ranges between 80 and 85% for both steps, the total yield from phenol increasing from 37% (anticipated and described in ES-A-412,484), to 70% as obtained with the method of this invention,
2) the purity of the obtained product. The epoxide obtained in the first step under mild reaction conditions has, in contrast to the one obtained with the known methods, such a purity grade that it can directly be used in the subsequent step and Carteolol can be obtained in a high yield and a high purity.
3) the method, in an industrial scale, is simpler in that the epoxidization reaction takes place at room temperature, with remarkable energy savings and with less complication and expense in the required devices. The work-up of the product is made easier and the purification procedures of the end product are simpler.

The following examples illustrate the invention.

### EXAMPLE 1

10 g of 5-hydroxy-3,4-dihydrocarbostyryl are suspended at the room temperature in 36 ml of water, 23 ml of dimethylformamide and 11 g of epichlorhydrine. Then 6.5 ml of 9.5 N sodium hydroxide are readily added. The obtained solution is stirred for 24 hours at room temperature and during this period the reaction product precipitates little by little. After filtration, 10.9 g of 5-(2,3-epoxy)-propoxy-3,4-dihydrocarbostyryl are obtained which corresponds to a yield of approximately 81%. The obtained product is readily reacted with about 35 g of tert-butylamine in 35 ml of dimethylsulphoxide, leaving the solution to react during about 10 hours at about 70°C. After the usual complementary treatment 13 g of Carteolol hydrochloride are obtained in an 80% yield, m.p. 278°C (ethanol).

### EXAMPLE 2

10 g of 5-hydroxy-3,4-dihydrocarbostyryl are suspended at room temperature in 36 ml of water, 12 ml of N-methylpyrrolidone and 11 g of epichlorhydrine. Then 6.5 ml of 9.5 N sodium hydroxide are readily added. The obtained solution is stirred for about 24 hours at room temperature; during this period the reaction product precipitates little by little. After filtration, 10.7 g of 5-(2,3-epoxy)-propoxy-3,4-dihydrocarbostyryl are obtained, which corresponds to an about 80% yield. The obtained product is readily reacted with 35 g of tert-butylamine in 35 ml of dimethylsulphoxide, leaving the solution to react for about 10 hours at approximately 70°C. After the usual complementary treatment, 12.8 g of Carteolol hydrochloride are obtained, in an about 80% yield, m.p. 277-278°C (ethanol).

### EXAMPLE 3

23.45 kg of 5-hydroxy-3,4-dihydrocarbostyryl are suspended at room temperature in 54 l of dimethylsulphoxide, 84 l of water and 22.7 kg of epichlorhydrine. Then 13 l of 9.5 N sodium hydroxide are added. The obtained solution is stirred for about 24 hours at room temperature; during this period the reaction product precipitates little by little. After filtration, 21.8 kg of 5-(2,3-epoxy)propoxy- 3,4-dihydrocarbostyryl are obtained, which corresponds to an about 80% yield. The resulting product is readily reacted with 70 kg of tert-butylamine in 70 l of dimethylsulphoxide, leaving the solution to react for about 10 hours at 70°C. After concentration, the base is crystallized from acetone, it is transformed into the hydrochloride and it is recrystallized from water, alcohol and ether. 26 kg of Carteolol hydrochloride are obtained, in an 80% yield, m.p. 278°C.

## Claims

1. A method for the preparation of 5-(3-tert-butylamino-2-hydroxypropoxy)-3,4-dihydrocarbostyryl hydrochloride of formula (I) 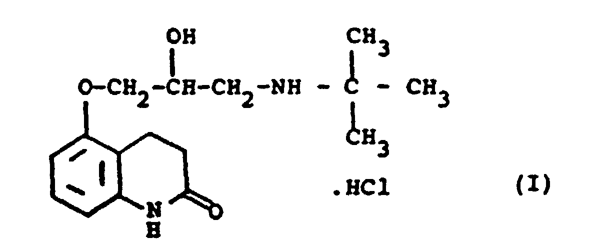 which process comprises:
a) reacting 5-hydroxy-3,4-dihydrocarbostyryl of formula (II) 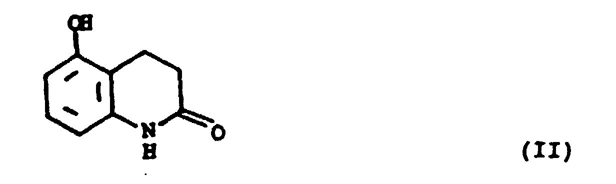 with epichlorhydrine of formula (III) 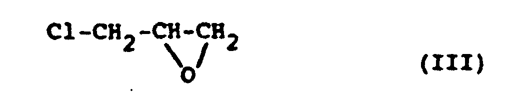
b) reacting the compound obtained in a) of formula (IV) 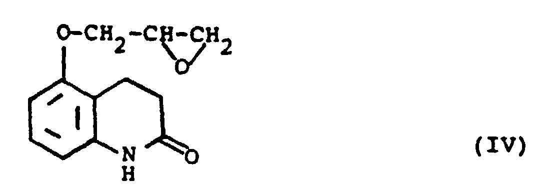 with tert-butylamine and
c) salifying the compound (I) to the corresponding hydrochloride.
charachterized in that:
- the reaction of step a) is carried out in a mixture of an aprotic solvent, selected from dimethylsulphoxide, dimethylformamide and methylpyrrolidone, with water at room temperature (15°-30°C), in the presence of alkali hydroxides, the solvent/water ratio ranging between 1:0.2 and 1:1;
- the reaction of step b) is carried out in the presence of dimethylsulphoxide.

2. A method according to claim 1 characterized in that said solvent:water ratio ranges between 1:0.3 and 1:0.6.

## Patentansprüche

1. Verfahren zur Herstellung von 5-(3-t-Butylamino-2-hydroxypropoxy)-3,4-dihydrocarbostyrylhydrochlorid der Formel (I) 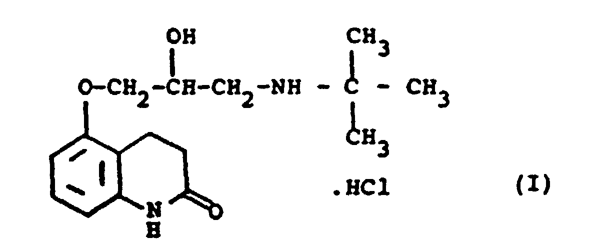 umfassend:
a) Umsetzen von 5-Hydroxy-3,4-dihydrocarbostyryl der Formel (II) 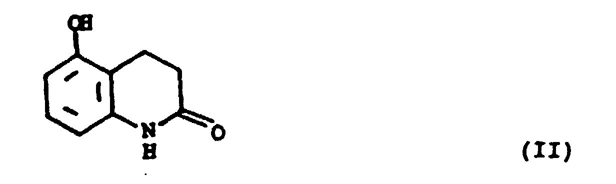 mit Epichlorhydrin der Formel (III) 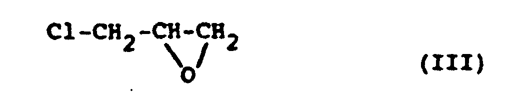
b) Umsetzen der in a) erhaltenen Verbindung der Formel (IV) 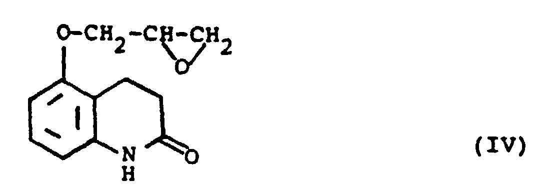 mit t-Butylamin und
c) Umsetzen der Verbindung (I) zum entsprechenden Hydrochlorid,
dadurch gekennzeichnet, daß:
- die Umsetzung in Stufe a) in einem Gemisch eines aprotischen Lösungsmittels, ausgewählt aus Dimethylsulfoxid, Dimethylformamid und Methylpyrrolidon, mit Wasser bei Raumtemperatur (15°-30°C) in Gegenwart von Alkalihydroxiden durchgeführt wird, wobei das Verhältnis von Lösungsmittel/Wasser im Bereich von 1:0,2 bis 1:1 liegt;
- die Umsetzung in Stufe b) in Gegenwart von Dimethylsulfoxid durchgeführt wird.

2. Verfahren nach Anspruch 1, dadurch gekennzeichnet, daß das Verhältnis von Lösungsmittel/Wasser im Bereich von 1:0,3 bis 1:0,6 liegt.

## Revendications

1. Procédé de préparation du chlorhydrate de 5-(3-tert-butylamino-2-hydroxypropoxy)-3,4-dihydrocarbostyryle de formule (I) 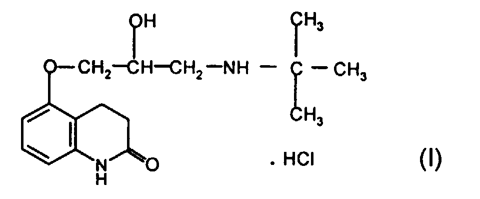 le procédé comprenant :
a) la mise en réaction de 5-hydroxy-3,4-dihydrocarbo-styryle de formule (II) 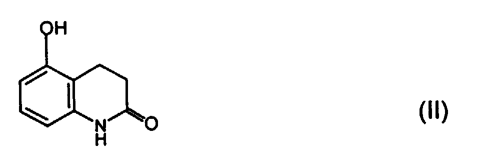 avec l'épichlorhydrine de formule (III) 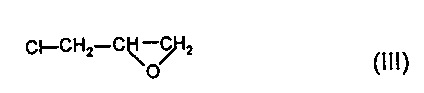
b) la mise en réaction du composé obtenu en a) de formule (IV) 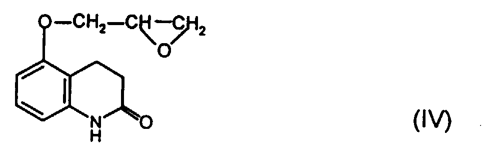 avec la tert-butylamine et
c) la salification du composé (I) pour obtenir le chlorhydrate correspondant,
caractérisé en ce que :
- la réaction de l'étape a) est mise en oeuvre dans un mélange d'un solvant aprotique, choisi parmi le diméthylsulfoxyde, le diméthylformamide et la méthylpyrrolidone, avec de l'eau, à température ambiante (15-30°C), en présence d'hydroxydes alcalins, le rapport solvant/eau étant dans la plage de 1:0,2 à 1:1 ;
- la réaction de l'étape b) est mise en oeuvre en présence de diméthylsulfoxyde.

2. Procédé selon la revendication 1, caractérisé en ce que ledit rapport solvant/eau est dans la plage de 1:0,3 à 1:0,6.1
